# EUROPEAN PATENT SPECIFICATION

(11) **EP 0 681 476 B1**
(45) Date of publication and mention of the grant of the patent: **27.08.1997**
(21) Application number: 92924415.0
(22) Date of filing: 16.11.1992
(51) Int. Cl.: A61K 31/60

(54) **UNIT DOSE FORM OF BISMUTH SUBSALICYLATE**
WISMUTSUBSALICYLAT IN FORM EINER EINHEITSDOSIS
PREPARATION PHARMACEUTIQUE DE DOSES UNITAIRES DE SUBSALICYLATE DE BISMUTH

(30) Priority: 22.11.1991 US 796193
(43) Date of publication of application: 15.11.1995
(73) Proprietor: THE PROCTER & GAMBLE COMPANY, Cincinnati, Ohio 45202 (US)
(72) Inventor: CRISANTI, Mark, Matthew, Cincinnati, OH 45249 (US); MURCIA, Daniel, Antonio, Fairfield OH 45014 (US)
(74) Representative: Engisch, Gautier
(86) International application number: US9209724
(87) International publication number: WO9309784

(56) References cited:
- DE-A- 3 220 765
- US-A- 1 764 933
- US-A- 4 588 589

## Description

### BACKGROUND OF THE INVENTION

The present invention is directed to pharmaceutical compositions in unit dosage form comprising bismuth subsalicylate and a source of chloride ions. The molar ratio of chloride ions to bismuth subsalicylate in these compositions is greater than 1:5 (chloride ions:bismuth subsalicylate).

Bismuth-containing pharmaceutical compositions are well known, being used widely to treat a variety of gastrointestinal disorders such as nausea, heartburn, and diarrhea. One such product is Pepto-Bismol® liquid and chewable tablets (sold by The Procter & Gamble Company). These products contain bismuth subsalicylate as the active ingredient. These and other bismuth-containing compositions are described generally in "Handbook of Nonprescription Drugs, 8th Ed." (American Pharmaceutical Association, Washington, D.C.; 1986), pages 73-74, and in the 1989 Physician's Desk Reference for Non-Prescription Drugs, 10th Edition (Medical Economics Company, Inc.; 1989), pages 642-3.

Notwithstanding the great effort already put forth to develop bismuth-containing compositions, there remains a continuing need to identify new bismuth-containing compositions. The bismuth subsalicylate-containing pharmaceutical compositions in unit dose form of the present invention are new, effective and convenient dosage forms for administering bismuth subsalicylate to the gastrointestinal tract.

Therefore, it is an object of the present invention to provide unit dosage forms of bismuth subsalicylate, preferably in a form suitable for swallowing such as caplets or tablets. A further object is to provide such compositions which readily provide the active bismuth subsalicylate ingredient to the gastrointestinal tract. An additional object is to provide a convenient, effective, portable unit dose form of bismuth subsalicylate for treating disturbances of the gastrointestinal tract.

These and other objects of the present invention will become readily apparent from the detailed description which follows.

All percentages used herein are by weight, and all ratios are molar ratios, unless otherwise specified.

### SUMMARY OF THE INVENTION

The present invention relates to unit dosage form pharmaceutical compositions comprising:
(a) from 5% to 99% of bismuth subsalicylate; and
(b) from 1% to 95% of a source of chloride ions; and wherein the molar ratio of chloride ion to bismuth subsalicylate is greater than 1:5, and wherein further said pharmaceutical composition is in unit dosage form.

The compositions of the present invention are useful in methods for treating or preventing disturbances of the gastrointestinal tract (e.g. diarrhea, gastritis, ulcers). These methods comprise orally administering to a human or lower animal in need of such treatment or prevention a safe and effective amount of a pharmaceutical composition of the present invention.

### DETAILED DESCRIPTION OF THE INVENTION

### Pharmaceutical Compositions:

The pharmaceutical compositions in unit dosage form of the present invention comprise the following components: (a) bismuth subsalicylate; (b) a source of chloride ion; and (c) optionally, pharmaceutically-acceptable carrier material.

These components, and the weight percentages and specific relative ratios for these components, are described in detail hereinafter.

### (a) Bismuth Subsalicylate:

Bismuth subsalicylate is a known pharmaceutical active agent. It is described, for example, in The Merck Index, 10th Edition (published by Merck & Co., Inc; 1983), No. 1284, "Bismuth Subsalicylate", page 180. A thorough review of bismuth subsalicylate as a therapeutic agent, including for example its chemistry, safety, mechanism of action, and pharmaceutical utilities, is also found in Reviews of Infectious Diseases, Vol. 12, Supplement 1, pages S1-S119 (1990).

The unit dosage compositions of the present invention typically comprise, by weight, from 5% to 99% of bismuth subsalicylate, preferably from 10% to 75%, and more preferably from 20% to 50%.

### (b) Source of Chloride Ion:

The term "source of chloride ion", as used herein, means any organic or inorganic chemical entity capable of providing a safe and effective amount of chloride ion in the stomach from the unit dosage form composition of the present invention. Preferred sources of chloride ion are inorganic salts of chloride ions, preferably selected from the group consisting of sodium chloride, magnesium chloride, calcium chloride, potassium chloride, and mixtures thereof. Most preferred is calcium chloride.

Compositions of the present invention typically comprise, by weight, from 1% to 95% of a source of chloride ions, preferably from 1% to 50%, and more preferably from 1% to 25%.

### (c) Molar Ratio of Chloride Ion to Bismuth Subsalicylate:

It has been discovered that the bioavailability of the bismuth subsalicylate (as measured by the level of salicylate present in the blood after administration of the unit dosage compositions) is substantially improved by including a source of chloride ions in the unit dosage form composition according to the present invention. This is desirable in order to minimize the time until hydrolysis of the bismuth subsalicylate; and to provide the fastest possible relief for the gastrointestinal disturbance being treated.

This benefit is particularly surprising in view of the normally inherently rich source of chloride ions in the stomach resulting from the hydrochloric acid generated in the stomach. However, while not intending to be limited by theory, it appears that the benefit achieved by including a source of chloride ion in the unit dosage composition is the result of providing the environment around the disintegrating unit dosage composition with an even higher concentation of chloride ion. This in turn is believed to accelerate the conversion of the bismuth subsalicylate to the bismuth oxychloride and salicylic acid in the stomach. Such a result is also believed to be highly desirable when food is present in the stomach and in those patients which have reduced hydrochloric acid production by the stomach, such as patients who are hypochlorhydric or achlorhydric.

The molar ratio of chloride ion to bismuth subsalicylate for purposes of the present invention is typically greater than 1:5, preferably within the range of from 1:5 to 10:1, more preferably within the range of from 1:2 to 5:1, and most preferably within the range of from 1:2 to 2:1.

### (d) Pharmaceutically-Acceptable Carrier Materials:

The oral pharmaceutical compositions herein may also optionally comprise one or more pharmaceutically-acceptable carrier materials. The term "pharmaceutically-acceptable carrier materials", as used herein, means one or more compatible solid or liquid filler diluents or encapsulating substances which are suitable for oral administration to a human or lower animal. The term "compatible", as used herein, means that the components of the oral pharmaceutical composition are capable of being commingled with the bismuth subsalicylate, and with each other, in a manner such that there is no interaction which would substantially reduce the pharmaceutical efficacy of the pharmaceutical composition under ordinary use situations. Pharmaceutically-acceptable carrier materials must, of course, be of sufficiently high purity and sufficiently low toxicity to render them suitable for oral administration to the human or lower animal being treated.

To the bismuth subsalicylate may be added any of the pharmaceutically-acceptable carrier materials known in the art for use in unit dosage forms, which are compatible with bismuth subsalicylate, such as:
- diluents, like lactose, starch, microcrystalline cellulose, sorbitol, mannitol, dibasic calcium phosphate dihydrate, calcium sulfate dihydrate, sucrose-based diluents, calcium carbonate, and mixtures thereof;
- binders, like acacia, cellulose derivatives, gelatin, glucose, polyvinylpyrrollidone, starch, sucrose, sorbitol, tragacanth, sodium alginate and mixtures thereof;
- disintegrants, like microcrystalline cellulose and cellulose derivatives, starch and its derivatives, alginic acid and its derivatives, ion-exchange resins, cross-linked sodium carboxymethyl cellulose, sodium starch glycolate, cross-linked polyvinylpyrrollidone and formaldehyde-caseine;
- lubricants, antiadherents and glidants, like magnesium-, calcium- and sodium stearates, stearic acid, hydrogenated castor oil, talc, water, polyethylene glycol, sodium lauryl sulfate, magnesium lauryl sulfate and silica. Other substances include sweetening agents (e.g., sugars such as lactose, glucose and sucrose, and non-nutritive sweeteners such as saccharin, aspartame, acesulfame, and cyclamate), coloring agents, flavoring agents, preservatives, and mixtures thereof.

Furthermore, the pharmaceutically-acceptable carrier materials may also comprise one or more auxiliary medicaments, preferably those which are intended to act in combination with it, such as non-steroidal anti-inflammatory compounds, H₂-antagonists, cytoprotectants (e.g., sucralfate), and synthetic prostaglandins. In particular the dosage units may contain antimicrobially effective medicaments such as antibiotics and chemotherapeutic compounds, more in particular medicaments effective against Campylobacter pylori (recently renamed Helicobacter pylori), such as the antimicrobially effective imidazoles, in particular metronidazole and tinidazole, penicillins, cephalosporins, tetracyclines, chinolones and macrolides. The dosage of the optionally present auxiliary medicaments will depend on the effectivity of the particular medicament used. Optional auxiliary medicaments useful herein are described in detail in: European Patent Application Publication No. 206,625, published December 30, 1986, by Marshall; and International Publication No. WO 86/05981, published October 23, 1986, by Borody.

The most preferred oral dosage units according to the present invention are caplets, tablets, and capsules, preferably of a size and shape suitable for swallowing. The dosage units according to the invention may be further coated.

Pharmaceutically-acceptable carrier materials typically comprise from 0% to 94%, preferably from 10% to 90%, and more preferably from 25% to 80%, by weight of the composition.

### Methods for Treating or Preventing Disturbances of the Gatrointestinal Tract:

Another aspect of the present invention is methods for treating or preventing disturbances of the gastrointestinal tract. Such methods comprised orally administering, to a human or lower animal in need of such treatment or prevention, a safe and effective amount of a pharmaceutical composition of the present invention.

The term "disturbances of the gastrointestinal tract", as used herein, encompasses any disease or other disorder of the gastrointestinal tract which is treatable or preventable by oral administration of a composition according to the present invention. Such disturbances are well known in the art, and include, for example: nausea; diarrhea, including the prevention of "travelers diarrhea" (as described, for example, in DuPont et al., "Prevention of Travelers' Diarrhea by the Tablet Formulation of Bismuth Subsalicylate", JAMA, 257, pages 1347-1350 (1987); heartburn; indigestion; upset stomach; and the treatment or prevention of gastritis and ulcers, especially when Campylobacter pylori (now known as Helicobacter pylori) infection is present (as described, for example, in European Patent Application Publication Numbers 206,626 and 206,627, published Dec. 30, 1986, by Marshall; and in McNulty et al., "Successful Therapy of Campylobacter Pyloridis Gastritis", Gastroenterology, 90, page 1547 (1986).

The phrase "safe and effective amount", as used herein, means an amount of a composition according to the present invention high enough to significantly and positively modify the condition to be treated or prevented, but low enough to avoid serious side effects (at a reasonable benefit/risk ratio), within the scope of sound medical judgment. The safe and effective amount of the composition will vary with the particular condition being treated, the age and physical condition of the patient being treated, the severity of the condition, the duration of treatment, the nature of concurrent therapy, the specific agent employed, the particular dose form utilized, and like factors within the knowledge and expertise of the attending physician.

The preferred daily dose of bismuth subsalicylate is within the range of from 100 milligrams of bismuth subsalicylate to 8500 milligrams of bismuth subsalicylate, and more preferably from 1000 milligrams to 6000 milligrams. Preferred individual doses of bismuth subsalicylate are from 100 milligrams to 2000 milligrams, with from 250 milligrams to 1500 milligrams being most preferred.

The following example further describes and demonstrates an embodiment within the scope of the present invention.

### EXAMPLE

### Swallowable Caplet

A swallowable unit dosage composition in the form of a tablet according to the present invention is prepared as follows:

| Component | Weight %** |
|---|---|
| Microcrystalline cellulose | 21.16 |
| Calcium carbonate | 21.15 |
| Bismuth subsalicylate* | 38.89 |
| Calcium chloride dihydrate(USP)* | 7.90 |
| Sodium starch glycolate | 6.00 |
| Povidone | 4.00 |
| Magnesium stearate | 0.80 |
| Dyes | 0.10 |

| | |
|---|---|
| *Molar ratio of chloride ion to bismuth subsalicylate is 1:1. | |
| **Dry weight basis; water is used during processing, and removed in processing until granulation has moisture content of 1-4%. | |

The caplets are prepared as follows. To a heated mixing vessel is added the calcium carbonate, half the dyes, and the microcrystalline cellulose. After thorough mixing, bismuth subsalicylate cake (50-55% water) is added with mixing and then most of the water is removed under vacuum. After cooling, the povidone, calcium chloride dihydrate and remaining dyes are mixed into the composition. Water is then added with mixing of the composition to form a granulate. The granulate is then dried (using heat and vacuum) to a moisture level of about 1-4%. The composition is then cooled, sodium starch glycolate is added and then mixed into the composition, followed by addition of the magnesium stearate and then mixing. The resulting granulate is screened and then compressed into 675 mg caplets having a hardness of 12-15 Strong Cobb units.

Ingestion by swallowing of two of these caplets by a person suffering from upset stomach induced by overindulgence of food and alcohol is effective for treating this gastrointestinal disturbance.

## Claims

1. A pharmaceutical composition in unit dosage form comprising:
(a) from 5% to 99% of bismuth subsalicylate; and
(b) from 1% to 95% of a source of chloride ions;
and wherein the molar ratio of chloride ion to bismuth subsalicylate is greater than 1:5, and wherein further said pharmaceutical composition is in unit dosage form.

2. The pharmaceutical composition according to Claim 1 wherein the source of chloride ions is selected from the group consisting of sodium chloride, magnesium chloride, calcium chloride, potassium chloride, and mixtures thereof.

3. The pharmaceutical composition according to Claim 2 wherein the unit dosage form is a swallowable dosage form.

4. The pharmaceutical composition according to Claim 1 wherein the source of chloride ions is calcium chloride.

5. The pharmaceutical composition according to Claim 2 wherein the molar ratio of chloride ion to bismuth subsalicylate is within the range of from 1:5 to 10:1.

6. A pharmaceutical composition in unit dosage form comprising:
(a) from 10% to 75% of bismuth subsalicylate; and
(b) from 1% to 50% of calcium chloride;
and wherein the molar ratio of chloride ion to bismuth subsalicylate is within the range of from 1:2 to 5:1, and wherein further said pharmaceutical composition is in unit dosage form.

7. The pharmaceutical composition according to Claim 6 wherein the unit dosage form is a swallowable dosage form.

8. A pharmaceutical composition in unit dosage form comprising:
(a) from 20% to 50% of bismuth subsalicylate;
(b) from 1% to 25% of calcium chloride; and
(c) from 25% to 80% pharmaceutically-acceptable carrier material;
and wherein the molar ratio of chloride ion to bismuth subsalicylate is within the range of from 1:2 to 2:1, and wherein further said pharmaceutical composition is in a swallowable unit dosage form of a caplet or tablet.

## Patentansprüche

1. Pharmazeutische Zusammensetzung in Einheitsdosisform, umfassend:
(a) von 5 % bis 99 % Bismutsubsalicylat; und
(b) von 1 % bis 95 % einer Chloridionenquelle;
und worin das Molverhältnis von Chloridion Zu Bismutsubsalicylat größer als 1:5 ist und worin weiterhin diese pharmazeutische Zusammensetzung in Dosiseinheitsform vorliegt.

2. Pharmazeutische Zusammensetzung nach Anspruch 1, worin die Chloridionenquelle aus der aus Natriumchlorid, Magnesiumchlorid, Calciumchlorid, Kaliumchlorid und Gemischen hievon bestehenden Gruppe ausgewählt ist.

3. Pharmazeutische Zusammensetzung nach Anspruch 2, worin die Dosiseinheitsform eine verschluckbare Dosierungsform ist.

4. Pharmazeutische Zusammensetzung nach Anspruch 1, worin die Chloridionenquelle Calciumchlorid ist.

5. Pharmazeutische Zusammensetzung nach Anspruch 2, worin das Molverhältnis von Chloridion zu Bismutsubsalicylat im Bereich von 1:5 bis 10:1 liegt.

6. Pharmazeutische Zusammensetzung in Einheitsdosisform, umfassend:
(a) von 10 % bis 75 % Bismutsubsalicylat; und
(b) von 1 % bis 50 % Calciumchlorid;
und worin das Molverhältnis von Chloridion zu Bismutsubsalicylat im Bereich von 1:2 bis 5:1 liegt und worin weiterhin diese pharmazeutische Zusammensetzung in Einheitsdosisform vorliegt.

7. Pharmazeutische Zusammensetzung nach Anspruch 6, worin die Einheitsdosisform eine verschluckbare Dosierungsform ist.

8. Pharmazeutische Zusammensetzung in Einheitsdosisform, umfassend:
(a) von 20 % bis 50 % Bismutsubsalicylat;
(b) von 1 % bis 25 % Calciumchlorid; und
(c) von 25 % bis 80 % pharmazeutisch annehmbares Trägermaterial;
und worin das Molverhältnis von Chloridion zu Bismutsubsalicylat im Bereich von 1:2 bis 2:1 liegt und worin weiterhin diese pharmazeutische Zusammensetzung in einer verschluckbaren Einheitsdosisform einer Pille oder Tablette vorliegt.

## Revendications

1. Composition pharmaceutique se présentant sous la forme de doses unitaires, comprenant :
(a) 5 à 99 % de sous-salicylate de bismuth, et
(b) 1 à 95 % d'une source d'ions chlorure,
et dans laquelle le rapport molaire des ions chlorure au sous-salicylate de bismuth est supérieur à 1:5 et par ailleurs ladite composition pharmaceutique se présente sous la forme de doses unitaires.

2. Composition pharmaceutique selon la revendication 1, dans laquelle la source d'ions chlorure est sélectionnée dans le groupe comprenant le chlorure de sodium, le chlorure de magnésium, le chlorure de calcium, le chlorure de potassium et leurs mélanges.

3. Composition pharmaceutique selon la revendication 2, dans laquelle la forme de dosage unitaire est une forme de dosage à avaler.

4. Composition pharmaceutique selon la revendication 1, dans laquelle la source d'ions chlorure est le chlorure de calcium.

5. Composition pharmaceutique selon la revendication 2, dans laquelle le rapport molaire des ions chlorure au sous-salicylate de bismuth se situe dans la plage de 1:5 à 10:1.

6. Composition pharmaceutique se présentant sous la forme de doses unitaires, comprenant :
(a) 10 à 75 % de sous-salicylate de bismuth, et
(b) 1 à 50 % de chlorure de calcium,
et dans laquelle le rapport molaire des ions chlorure au sous-salicylate de bismuth se situe dans la plage de 1:2 à 5:1 et par ailleurs ladite composition pharmaceutique se présente sous la forme de doses unitaires.

7. Composition pharmaceutique selon la revendication 6, dans laquelle la forme de dosage unitaire est une forme de dosage à avaler.

8. Composition pharmaceutique se présentant sous la forme de doses unitaires, comprenant :
(a) 20 à 50 % de sous-salicylate de bismuth,
(b) 1 à 25 % de chlorure de calcium, et
(c) 25 à 80 % d'un véhicule pharmaceutiquement acceptable,
et dans laquelle le rapport molaire des ions chlorure au sous-salicylate de bismuth se situe dans la plage de 1:2 à 2:1 et par ailleurs ladite composition pharmaceutique se présente sous la forme de doses unitaires à avaler formées de capsules ou de comprimés.
